# EUROPÄISCHE PATENTSCHRIFT

(11) **EP 3 747 410 B1**
(45) Veröffentlichungstag und Bekanntmachung des Hinweises auf die Patenterteilung: **27.12.2023**
(21) Anmeldenummer: 19178017.0
(22) Anmeldetag: 04.06.2019
(51) Int. Cl.: A61F 7/00, H05B 3/26

(54) **VORRICHTUNG UND SYSTEM ZUR THERMISCH-MEDIZINISCHEN BEHANDLUNG EINER HAUTOBERFLÄCHE EINES MENSCHEN**
DEVICE AND SYSTEM FOR THERMAL MEDICAL TREATMENT OF A SKIN SURFACE OF A HUMAN
DISPOSITIF ET SYSTÈME DE TRAITEMENT THERMIQUE MÉDICAL D'UNE SURFACE DE LA PEAU D'UN HOMME

(43) Veröffentlichungstag der Anmeldung: 09.12.2020
(73) Patentinhaber: Kamedi GmbH, 76131 Karlsruhe (DE)
(72) Erfinder: Liedtke, Lukas Heinrich, 34497 Korbach (DE); Meyer, Armin, 13585 Berlin (DE); Hotz, Stefan, 69242 Mühlhausen (DE); Reuter, Christof, 77948 Friesenheim (DE)
(74) Vertreter: Durm Patentanwälte PartG mbB

(56) Entgegenhaltungen:
- WO-A1-2019/020144
- KR-A- 20130 093 339
- US-A1- 2012 065 556
- US-A1- 2012 209 357
- US-A1- 2013 053 733

## Beschreibung

Die vorliegende Erfindung betrifft eine Vorrichtung zur thermisch-medizinischen Behandlung einer Hautoberfläche eines Menschen. Die vorliegende Erfindung betrifft weiterhin ein System und ein Verfahren zur thermisch-medizinischen Behandlung einer Hautoberfläche eines Menschen.

Hautirritationen, die beispielsweise durch Insektenstiche, Pflanzen, Fische, Algen, Quallen oder andere Nesseltiere verursacht werden, können durch eine Wärmebehandlung kuriert bzw. in ihren Auswirkungen gelindert werden. Möglichst kurzfristig nach Auftreten der Hautirritation wird die betreffende Hautpartie erwärmt, beispielsweise auf eine Temperatur zwischen 45 und 65 Grad Celsius. Durch die Erwärmung wird die Durchblutung angeregt und eine Hautreaktion, die zu einer Hautirritation führen kann, unterdrückt. Schmerz und Juckreiz werden gelindert.

Dadurch, dass die oben genannten Ursachen derartiger Hautirritationen oft im Outdoor-Bereich auftreten, sind zwischenzeitlich portable Vorrichtungen zum Durchführen einer entsprechenden Wärmebehandlung verfügbar. Diese werden normalerweise über eine Batterie betrieben und erlauben oft die Einstellung von Behandlungsparametern, insbesondere einer Behandlungstemperatur und einer Behandlungsdauer. Hierbei hat es sich als vorteilhaft herausgestellt, eine Vorrichtung zum Ankoppeln an ein Mobilgerät zu verwenden, wobei ein Akku des Mobilgeräts für die Energieversorgung der Vorrichtung und das Mobilgerät selbst zur Einstellung der Parameter verwendet wird.

In der WO 2019/020144 A1 wird in diesem Zusammenhang eine koppelbare portable Vorrichtung zur thermisch-medizinischen Behandlung der Haut offenbart. Die Vorrichtung umfasst einen Behandlungsaufsatz, der über eine Schnittstelle mit einer mobilen Datenverarbeitungseinheit, vorzugsweise einem Smartphone oder einem Tablet, koppelbar ist. Im gekoppelten Zustand ist ein den Behandlungsaufsatz übergreifendes Gehäuse des Behandlungsaufsatzes unmittelbar unterhalb der von der Datenverarbeitungseinheit abgewandten Oberfläche mit einem Heizelement versehen. Das Heizelement ist mit einem Temperatursensor gekoppelt. Das Heizelement ist unter Zwischenschaltung einer Steuereinheit mit einer elektrisch leitenden Schnittstelle oder über eine Bluetooth-Verbindung mit der Datenverarbeitungseinheit derart verbunden, dass das Heizelement über einen oder mehrere in die Datenverarbeitungseinheit integrierte Akkumulatoren so mit Strom versorgt ist, dass das Heizelement auf eine über die Steuerungseinheit vorgegebene Temperatur erwärmbar ist.

Die US 2012/0209357 A1 betrifft eine portable Vorrichtung zum Behandeln der Haut mittels Licht und Wärme sowie ein Verfahren zum Steuern der Vorrichtung. Das Verfahren umfasst das Erwärmen einer Heizeinheit zum Vorbereiten einer Erwärmung einer Spitze des Behandlungsgeräts auf eine vordefinierte Temperatur, wenn ein Kontrollknopf des Behandlungsgeräts gedrückt wird. Weiterhin umfasst das Verfahren einen Schritt des Anschaltens einer Lichtquelle, die innerhalb des Gehäuses der Behandlungsvorrichtung angeordnet ist, und des Ausführens einer Bestrahlungsbehandlung der Haut durch die Spitze zusammen mit der Hitze, wenn die Erwärmung der Spitze abgeschlossen ist.

Die US 2013/0053733 A1 betrifft Verfahren und Systeme zum Behandeln einer Fehlfunktion einer Meibom-Drüse. Das Verfahren umfasst einen Schritt des Einleitens einer Hochfrequenzenergie zu einem internen Teil einer Meibom-Drüse. Das Verfahren umfasst weiterhin einen Schritt des selektiven Abzielens auf einen Verschluss eines Gangs in der Meibom-Drüse mit der applizierten Hochfrequenzenergie zum Aufschmelzen, Lösen oder Aufweichen des Verschlusses. Zudem wird eine Vorrichtung zum Behandeln einer Fehlfunktion der Meibom-Drüse offenbart. Die Vorrichtung umfasst zumindest eine Hochfrequenzelektrode, die zum Zuführen einer Hochfrequenzenergie zu einem internen Teil einer Meibom-Drüse ausgebildet ist.

Die US 2012/0065556 A1 betrifft ein Verfahren und eine Vorrichtung zum Stimulieren der Meibom-Drüsen des Augenlids. Die Vorrichtung umfasst einen Griff und einen Kopf, der nicht abnehmbar und mit dem Griff integriert ausgebildet ist. Der Kopf umfasst ein Augenteil, das oszilliert, um eine Massagefunktion an einem Augenlid auszuführen. Das Augenteil umfasst ein vorderes Ende, das konkav ausgebildet ist, um über ein Augenlid zu passen. Die Vorrichtung umfasst weiterhin eine Heizeinheit, die innerhalb des Augenteils angeordnet ist, und einen Temperatursensor, der innerhalb des Augenteils angeordnet ist.

Bei solchen Vorrichtungen besteht eine Herausforderung darin, einerseits eine effiziente Erwärmung zu gewährleisten und andererseits eine effiziente Herstellbarkeit zu ermöglichen. Die verwendeten Heizelemente sind oft Spezialbauteile mit vergleichsweise hohen Stückkosten. Zudem ist die verfügbare Energie oft begrenzt. Ein Ziel ist es daher, ein minimal dimensioniertes Heizelement so anzuordnen, dass möglichst viel der erzeugten Hitzemenge der zu behandelnden Hautoberfläche zugeführt werden kann.

Ausgehend hiervon stellt sich der vorliegenden Erfindung die Aufgabe, eine effizient herstellbare Vorrichtung zur thermisch-medizinischen Behandlung einer Hautoberfläche eines Menschen zu schaffen. Insbesondere sollen der Hardwareaufwand durch einen effizienten Wärmetransport zwischen einem Heiz element und der Hautoberfläche reduziert werden und eine kosteneffizient fertigbare Vorrichtung geschaffen werden.

Zum Lösen dieser Aufgabe betrifft die vorliegende Erfindung in einem Aspekt eine Vorrichtung zur thermisch-medizinischen Behandlung einer Hautoberfläche eines Menschen, mit:
- einer Anwendungsfläche mit einer Vorderseite zum Anlegen an die Hautoberfläche; und
- einer Leiterplatte mit einem darauf angeordneten Heizelement zum Erwärmen der Anwendungsfläche basierend auf einer Versorgungsspannung, wobei
- die Anwendungsfläche mit einer Rückseite an einer Stirnseite der Leiterplatte in einem Winkel zu der Leiterplatte angeordnet ist; und
- das Heizelement in einem Bereich der der Anwendungsfläche zugewandten Stirnseite der Leiterplatte auf der Leiterplatte angeordnet ist und mit der Anwendungsfläche thermisch gekoppelt ist,
- wobei das Heizelement an einem Rand der Leiterplatte angeordnet ist.

In einem weiteren Aspekt betrifft die vorliegende Erfindung ein System zur thermisch-medizinischen Behandlung einer Hautoberfläche eines Menschen, mit:
- einer Vorrichtung wie zuvor beschrieben; und
- einem Mobilgerät mit einer Geräteschnittstelle zum reversiblen Ankoppeln der Vorrichtung und zum Versorgen der Vorrichtung mit der Versorgungsspannung, wobei
- die Geräteschnittstelle vorzugsweise als Micro-USB, USB-C oder Lightning Anschluss ausgebildet ist.

In einem weiteren Aspekt betrifft die vorliegende Erfindung ein Verfahren zur thermisch-medizinischen Behandlung einer Hautoberfläche eines Menschen, mit den Schritten:
- Empfangen einer Nutzereingabe eines Nutzers eines Mobilgeräts;
- Ermitteln einer gewünschten Behandlungstemperatur zum Anlegen an die Hautoberfläche;
- Erzeugen von Steuerdaten basierend auf der gewünschten Behandlungstemperatur; und
- Übermitteln der Steuerdaten an eine Vorrichtung wie zuvor beschrieben, die an das Mobilgerät angekoppelt ist.

Ein weiterer Aspekt der Erfindung betrifft ein entsprechend dem Verfahren ausgebildetes Computerprogrammprodukt mit Programmcode zum Durchführen der Schritte des Verfahrens, wenn der Programmcode auf einem Computer ausgeführt wird, sowie ein Speichermedium, auf dem ein Computerprogramm gespeichert ist, das, wenn es auf einem Computer ausgeführt wird, eine Ausführung des hierin beschriebenen Verfahrens bewirkt.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere können das System, das Verfahren sowie das Computerprogrammprodukt entsprechend der für die Vorrichtung in den abhängigen Ansprüchen beschriebenen Ausgestaltungen ausgeführt sein.

Erfindungsgemäß ist es vorgesehen, dass ein Heizelement im Bereich einer Stirnseite einer Leiterplatte auf der Leiterplatte angeordnet ist. Im Winkel zur Leiterplatte ist eine Anwendungsfläche angeordnet, die mit ihrer Vorderseite in Kontakt mit der zu behandelnden Hautpartie des Menschen bringbar ist. Durch das Heizelement wird über eine thermische Kopplung die Anwendungsfläche erwärmt. Über die Anwendungsfläche kann die Wärme an die Hautoberfläche weitergegeben werden.

Im Vergleich zu bisherigen Ansätzen ergibt sich durch die erfindungsgemäße Anordnung eine verbesserte, effiziente und verlustarme thermische Kopplung zwischen Heizelement und Anwendungsfläche, die kosteneffizient herstellbar ist. In bisherigen Ansätzen ist die Heizfläche zumeist parallel zu der Leiterplatte angeordnet. Unterschiedlich hohe Elemente auf der Leiterplatte (Sicherungselemente, Überwachungselemente, Transistoren, Heizelemente etc.) resultieren dabei in einer schlechteren thermischen Kopplung zwischen den Elementen und der Anwendungsfläche. Zu einem ebensolchen Effekt führt auch ein Aufschwimmen der verwendeten Bauelemente in einem Lötprozess, wodurch eine Toleranzkette vergrößert wird und eine Wärmeübertragung erschwert wird. Es wird ein größeres Volumen an Wärmeleitmedium erforderlich, wodurch eine Nutzleistung und Dynamik verschlechtert werden. Zudem ist die Orientierung sowie die Ausdehnung der Anwendungsfläche eingeschränkt, sodass die Designmöglichkeiten hinsichtlich der Form des Gesamtsystems reduziert werden. Durch die erfindungsgemäße Anordnung werden diese Nachteile ausgeräumt.

Im Vergleich zu bisherigen Ansätzen, bei denen eine Verkabelung zwischen Heizelement und Anwendungsfläche und/oder ein direktes Aufbringen des Heizelements auf die Anwendungsfläche vorgesehen waren, ergibt sich zudem eine wesentlich vereinfachte Herstellbarkeit. In der Herstellung kann ein automatisches Bestückungsverfahren eingesetzt werden. Hierdurch können Kosten reduziert werden. Zudem sind die Ausgestaltung und die Orientierung des Heizelements weitgehend frei wählbar, was insbesondere im Rahmen einer Miniaturisierung von Produkten vorteilhaft ist. Dadurch, dass lediglich die beheizten Flächen bzw. das Heizelement direkten Kontakt zur Anwendungsfläche haben und zudem die Leiterplatte vergleichsweise klein dimensioniert sein kann, wird die Trägheit des Gesamtsystems auf ein Minimum reduziert.

In einer bevorzugten Ausgestaltung ist das Heizelement an einem Rand der Leiterplatte angeordnet. Alternativ oder zusätzlich beträgt der Winkel zwischen Leiterplatte und Anwendungsfläche 90 Grad. Durch die Anordnung des Heizelements direkt am Rand der Leiterplatte wird eine gute thermische Kopplung zwischen Anwendungsfläche und Heizelement bei geringen Verlusten erreicht.

Minimal dimensionierte Heizelemente können verwendet werden. Eine effiziente Herstellbarkeit sowie geringe Materialkosten werden erreicht. Durch die Anordnung in einem Winkel von 90 Grad ist eine einfache Herstellbarkeit gewährleistet.

In einer bevorzugten Ausgestaltung ist ein erster Kontaktierungsbereich (Lötpad) des Heizelements auf der Leiterplatte der Anwendungsfläche zugewandt und ein zweiter Kontaktierungsbereich des Heizelements auf der Leiterplatte der Anwendungsfläche abgewandt. Hierbei ist der erste Kontaktierungsbereich vorzugsweise kleiner als der zweite Kontaktierungsbereich. Die Anordnung am Rand der Leiterplatte bewirkt eine optimierte Wärmeleitung. Durch die unterschiedliche Dimensionierung kann ausgenutzt werden, dass sich die SMD-Bauteile in einem Lötvorgang automatisch an den Kontaktierungsbereichen (Lötpads) ausrichten. Eine Positionierung direkt am Rand der Leiterplatte verbessert die Wärmeleitung zur Anwendungsfläche.

In einer bevorzugten Ausgestaltung umfasst das Heizelement einen Heizwiderstand. Eine Ausdehnung des Heizwiderstands in einer Länge zwischen zwei Kontaktierungsseiten ist vorzugsweise kleiner als eine Ausdehnung in einer Breite des Heizelements orthogonal zu der Länge. Es wird ein Widerstand verwendet, der vergleichsweise breit ist, um hierdurch eine optimierte Wärmeleitung an die Anwendungsfläche sicherzustellen. Der Wärmeverlust wird reduziert.

In einer bevorzugten Ausgestaltung liegt die Anwendungsfläche an der Stirnseite der Leiterplatte an. Die Stirnseite der Leiterplatte ist vorzugsweise metallisiert. Durch ein Anlegen der Anwendungsfläche an die Stirnseite wird eine einfache Herstellbarkeit erreicht. Der Zusammenbau wird vereinfacht. Durch die Metallisierung der Stirnseite kann eine weiter verbesserte Wärmeleitung durch eine vergrößerte Fläche ermöglicht werden.

In einer bevorzugten Ausgestaltung umfasst die Vorrichtung eine Energieschnittstelle zum reversiblen Ankoppeln der Vorrichtung an ein Mobilgerät und zum Empfangen der Versorgungsspannung von dem Mobilgerät. Die Energieschnittstelle ist vorzugsweise als Mikro-USB, USB-C oder Lightning-Stecker ausgebildet und der Stecker ist sehr bevorzugt orthogonal zur Anwendungsfläche ausgerichtet. Die Vorrichtung ist an ein Mobilgerät koppelbar und kann dessen Energieversorgung verwenden. Hierdurch wird die Portabilität der Vorrichtung sichergestellt. Es ist nicht erforderlich, dass die Vorrichtung einen eigenen Energiespeicher umfasst, der gegebenenfalls gepflegt bzw. geladen werden müsste. Ein orthogonal ausgerichteter Stecker wird beim Andrücken der Anwendungsfläche an die Hautoberfläche nicht mit Scher- und Biegekräften beaufschlagt, die zu Materialdefekten führen können. Die auf die entsprechende Schnittstelle des Mobilgeräts wirkenden Kräfte werden ebenfalls vermindert.

In einer bevorzugten Ausgestaltung umfasst das Heizelement mindestens zwei Teil-Heizelemente, die vorzugsweise identisch sind. Die mindestens zwei Teil-Heizelemente sind weiter vorzugsweise jeweils einzeln ansteuerbar, um ein Temperaturprofil an die Anwendungsfläche anzulegen. Durch die Verwendung mehrerer Teil-Heizelemente kann ein Temperaturprofil angelegt werden, in dem die mehreren Teil-Heizelemente mit unterschiedlichen Temperaturen angesteuert werden. Hierdurch kann beispielsweise ein zentraler Bereich der Hautirritation mit einer höheren Temperatur behandelt werden als ein außenliegender Bereich. Es wird eine verbesserte Behandlung möglich.

In einer bevorzugten Ausgestaltung ist ein erstes Teil-Heizelement auf einer ersten Seite der Leiterplatte angeordnet. Ein zweites Teil-Heizelement ist auf einer zweiten Seite der Leiterplatte angeordnet. Das erste Teil-Heizelement und das zweite Teil-Heizelement sind vorzugsweise in ihrer Position entlang der Stirnseite der Leiterplatte versetzt angeordnet. Durch eine beidseitige Anordnung von Heizelementen auf der Leiterplatte kann ein verbesserter Wärmetransport auf die Anwendungsfläche ermöglicht werden. Die Wärmeverluste werden minimiert. Durch eine versetzte Anordnung aus Sicht der Anwendungsfläche wird eine möglichst gleichmäßige Temperaturverteilung über die Anwendungsfläche erreicht. Hierdurch kann der Behandlungserfolg bzw. der Effekt der Wärmebehandlung gesteigert werden.

In einer bevorzugten Ausgestaltung sind ein unteres Teil-Heizelement und ein oberes Teil-Heizelement aufeinandergestapelt auf der Leiterplatte angeordnet. Durch eine aufeinanderliegende Anordnung ergibt sich eine weiter verbesserte Wärmeleitung zwischen Heizelement und Anwendungsfläche. Die Anwendungsfläche wird über einen größeren Bereich mit Wärme beaufschlagt. Wärmeverluste werden vermindert.

In einer bevorzugten Ausgestaltung umfasst die Leiterplatte eine Aussparung auf einer der Anwendungsfläche abgewandten Seite des Heizelements, um einen Wärmeabfluss zu vermindern. Zwar ist die Wärmeübertragung durch ein nichtmetallisiertes PCB (Leiterplatte) gering. Durch eine Aussparung kann diese jedoch weiter reduziert werden. Es wird eine optimierte Energieausnutzung erreicht.

In einer bevorzugten Ausgestaltung umfasst die Vorrichtung ein auf der Leiterplatte angeordnetes Messelement zum Ermitteln einer Temperatur der Anwendungsfläche. Das Messelement ist in einem Bereich der der Anwendungsfläche zugewandten Stirnseite der Leiterplatte auf der Leiterplatte angeordnet und mit der Anwendungsfläche thermisch gekoppelt. Vorzugsweise umfasst das Messelement einen Temperatursensor. Ein Messelement erlaubt eine genaue Steuerung der Temperatur der Anwendungsfläche. Es wird eine Rückkopplung ermöglicht, um sicherzustellen, dass es nicht zu Verletzungen der Hautoberfläche kommt. Zudem kann eine Prozesskontrolle realisiert werden. Beispielsweise kann ein Temperaturprofil nach einer Vorgabe abgefahren werden.

In einer bevorzugten Ausgestaltung umfasst die Vorrichtung ein auf der Leiterplatte angeordnetes Sicherungselement zum Überwachen einer Funktion des Heizelements. Das Sicherungselement umfasst vorzugsweise eine selbstrückstellende Sicherung oder einen selbstregulierenden Schaltkreis auf Basis von beispielsweise temperaturabhängigen Widerständen oder auch eine Schmelzsicherung. So wird sichergestellt, dass es nicht zu einer Verletzung des Anwenders kommt, wenn das Heizelement beispielsweise aufgrund einer Fehlfunktion übermäßig erwärmt wird. Die Sicherheit des Anwenders wird verbessert.

In einer bevorzugten Ausgestaltung umfasst die Vorrichtung ein auf der Leiterplatte angeordnetes Steuerelement zum Steuern des Heizelements. Das Steuerelement umfasst vorzugsweise einen Transistor zum Schalten der Versorgungsspannung. Insbesondere kann es ausreichend sein, die Versorgungsspannung an- und abzuschalten, um durch eine entsprechende Anlegedauer eine gewünschte Temperatur des Heizelements zu erreichen. Es ergibt sich eine kostengünstige Realisierbarkeit.

In einer bevorzugten Ausgestaltung umfasst die Vorrichtung eine auf der Leiterplatte angeordnete Prozessoreinheit zum Ansteuern des Heizelements, um eine Behandlungstemperatur der Anwendungsfläche einzustellen. Alternativ oder zusätzlich ist es möglich, durch eine Prozessoreinheit eine Behandlungstemperatur vorzugeben. Eine präzise Steuerung der Vorrichtung wird ermöglicht.

In einer bevorzugten Ausgestaltung umfasst die Vorrichtung eine auf der Leiterplatte angeordnete Prozessoreinheit zum Ansteuern des Heizelements, um eine Behandlungstemperatur der Anwendungsfläche einzustellen. Weiterhin umfasst die Vorrichtung eine Steuerschnittstelle zum Empfangen von Steuerdaten mit Informationen über eine gewünschte Behandlungstemperatur, wobei die Prozessoreinheit zum Ansteuern des Heizelements basierend auf den Steuerdaten ausgebildet ist. Die Steuerschnittstelle ist vorzugsweise zum Empfangen der gewünschten Behandlungstemperatur von einer App eines Mobilgeräts ausgebildet. Es ist möglich, dass eine Prozessoreinheit auf der Leiterplatte eine Steuerung der Erwärmung bzw. der Behandlung basierend auf einer Vorgabe einer Mobilgeräte-App vornimmt. Steuerdaten werden von der App an die Prozessoreinheit übertragen. Die Prozessoreinheit steuert dann die Temperierung der Anwendungsfläche. Es ergibt sich eine gute Bedienbarkeit und ein gesteigerter Behandlungseffekt.

In einer bevorzugten Ausgestaltung umfasst die Leiterplatte ein erstes Befestigungselement und die Anwendungsfläche ein zweites Befestigungselement, um die Anwendungsfläche an der Leiterplatte zu befestigen. Eines der Befestigungselemente ist als Haken ausgebildet und das andere Befestigungselement ist als Aussparung ausgebildet, in die der Haken zum Befestigen der Anwendungsfläche in der Leiterplatte eingreifen kann. Eine Haken-Aussparung-Verbindung zwischen Leiterplatte und Anwendungsfläche ermöglicht eine einfache Herstellbarkeit und insbesondere Montage. Zudem wird es ermöglicht, bei Bedarf einen Austausch eines der beiden Elemente vorzunehmen. Eine kostengünstige Umsetzung wird erreicht.

In einer weiteren Ausführung umfasst die Vorrichtung ein Wärmeleitmedium zwischen Heizelement und Anwendungsfläche. Durch ein Wärmeleitmedium wird eine weiter optimierte Wärmeleitung ermöglicht.

Das erfindungsgemäße Verfahren kann beispielsweise in Software implementiert sein und in Form einer Mobilgeräte-App auf einem Mobilgerät ausgeführt werden. Über das erfindungsgemäße Verfahren kann eine Ansteuerung der zuvor beschriebenen Vorrichtung erfolgen. Ein Nutzer eines Mobilgeräts kann Parameter vorgeben, um eine auf die jeweiligen Bedürfnisse optimierte Ansteuerung bzw. Behandlungstemperatur einzustellen. Hierbei kann basierend auf Benutzereingaben eine automatisierte Ermittlung einer notwendigen Behandlungstemperatur erfolgen.

Unter einer Hautoberfläche eines Menschen versteht sich eine Hautpartie des Menschen. Insbesondere kann eine Hautoberfläche eine Hautpartie in der Größe ungefähr eines Quadratzentimeters bezeichnen. Eine Anwendungsfläche ist insbesondere aus einem biokompatiblen Material gefertigt. Eine Leiterplatte ist insbesondere ein PCB. Unter einer thermischen Kopplung versteht sich eine wärmeleitende Anbindung.

Die Erfindung wird nachfolgend anhand einiger ausgewählter Ausführungsbeispiele im Zusammenhang mit den beiliegenden Zeichnungen näher beschrieben und erläutert. Es zeigen:
- Figur 1: eine schematische Darstellung eines erfindungsgemäßen Systems zur thermisch-medizinischen Behandlung einer Hautoberfläche eines Menschen;
- Figur 2: eine schematische Darstellung einer erfindungsgemäßen Vorrichtung;
- Figur 3: eine schematische Darstellung einer Anordnung der Elemente in einer erfindungsgemäßen Vorrichtung;
- Figur 4: eine schematische Darstellung einer Vorrichtung mit einem Steuerungselement;
- Figur 5: eine schematische Darstellung einer erfindungsgemäßen Vorrichtung in einer Schnittansicht orthogonal zu der Leiterplatte im Bereich der Stirnseite;
- Figur 6: eine schematische Darstellung einer bevorzugten Ausführung des Heizelements;
- Figur 7: eine schematische Darstellung einer erfindungsgemäßen Vorrichtung mit vier Teil-Heizelementen;
- Figur 8: eine schematische Darstellung einer erfindungsgemäßen Vorrichtung mit einer Aussparung;
- Figur 9: eine schematische Darstellung einer erfindungsgemäßen Vorrichtung mit mehreren aufeinandergestapelten Teil-Heizelementen;
- Figur 10: eine schematische perspektivische Darstellung des Kopplungsbereichs zwischen Anwendungsfläche und Leiterplatte;
- Figur 11: eine schematische Darstellung alternativ ausgebildeter Heizelemente;
- Figur 12: eine schematische Darstellung eines Kopplungsmechanismus zwischen Leiterplatte und Anwendungsfläche;
- Figur 13: eine schematische Anordnung der Elemente auf der Leiterplatte; und
- Figur 14: eine schematische Darstellung eines erfindungsgemäßen Verfahrens.

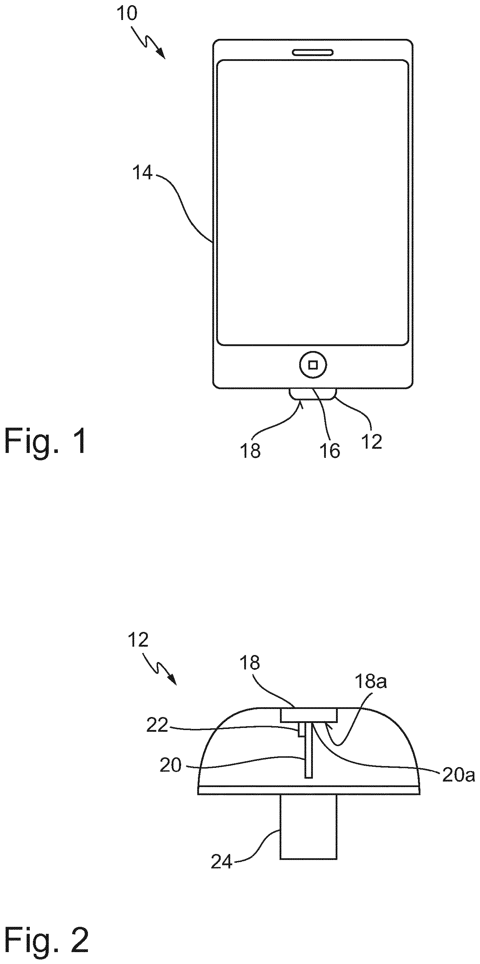

In der Fig. 1 ist schematisch ein erfindungsgemäßes System zur thermisch-medizinischen Behandlung einer Hautoberfläche eines Menschen dargestellt. Das System 10 umfasst eine Vorrichtung 12 sowie ein Mobilgerät 14, wobei das Mobilgerät eine Geräteschnittstelle 16 aufweist, an die die Vorrichtung 12 angekoppelt ist. Insbesondere kann die Geräteschnittstelle 16 als Mikro-USB, USB-C oder Lightningbuchse ausgebildet sein. Um eine Wärmebehandlung von Hautirritationen zu ermöglichen, umfasst die Vorrichtung 12 eine erwärmbare Anwendungsfläche 18 zum Anlegen an die Hautoberfläche im Bereich der Hautirritation.

In der Fig. 2 ist die erfindungsgemäße Vorrichtung 12 schematisch in vergrößerter Ansicht dargestellt. Die Vorrichtung 12 umfasst die Anwendungsfläche 18 und eine Leiterplatte 20 mit einem darauf angeordneten Heizelement 22. Die Anwendungsfläche 18 ist dazu ausgebildet, an eine Hautoberfläche eines Menschen angelegt zu werden (nicht dargestellt). Die erfindungsgemäße Vorrichtung 12 umfasst im dargestellten Ausführungsbeispiel weiterhin eine Energieschnittstelle 24, die als Stecker ausgebildet ist und mit der Geräteschnittstelle des Mobilgeräts koppelbar. Über die Energieschnittstelle kann die Vorrichtung 12 mit Energie und/oder mit Steuerbefehlen versorgt werden. Die Anwendungsfläche 18 ist im dargestellten Beispiel senkrecht zur Energieschnittstelle 24 ausgerichtet, sodass ein Anwender die Anwendungsfläche 18 an die Hautoberfläche anpressen kann.

Über das Heizelement 22 wird die Anwendungsfläche 18 erwärmt. Hierzu ist das Heizelement 22 mit der Anwendungsfläche 18 thermisch gekoppelt. Die Anwendungsfläche 18 ist mit einer Rückseite 18a an einer Stirnseite 20a der Leiterplatte 20 in einem Winkel zu der Leiterplatte 20 angeordnet. Im dargestellten Ausführungsbeispiel beträgt der Winkel 90 Grad. Das Heizelement 22 ist vorzugsweise ein SMD-Heizwiderstand, der in einem vollautomatisierbaren Bestückungsprozess auf die Leiterplatte 20 aufgebracht werden kann. Das Heizelement wird bestromt, sodass Hitze entsteht. Diese Hitze wird über die Anwendungsfläche 18 an die Hautoberfläche des Menschen angelegt. Um einen möglichst effizienten Montageprozess und eine gute thermische Kopplung zu ermöglichen, ist es erfindungsgemäß vorgesehen, das Heizelement 22 an der Stirnseite 20a der Leiterplatte 20 anzuordnen.

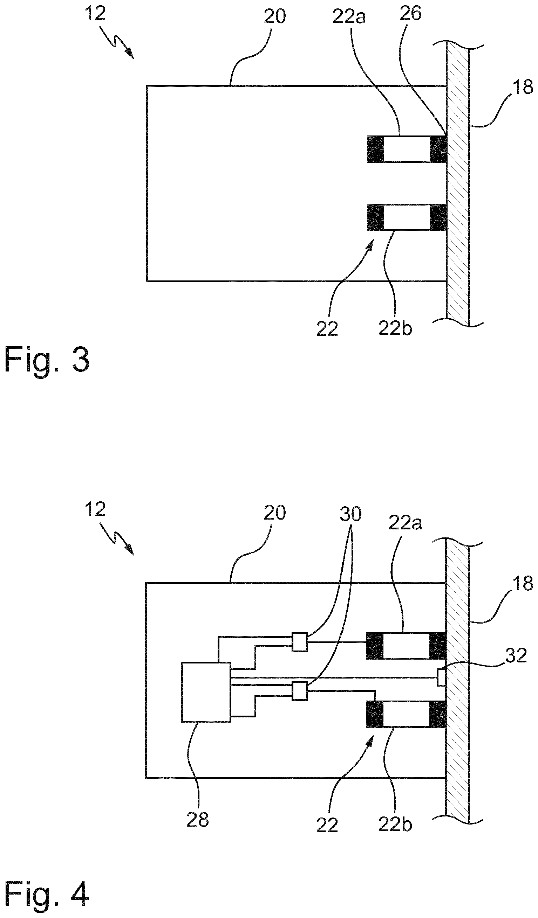

In der Fig. 3 ist eine schematische Draufsicht auf die Leiterplatte 20 der Vorrichtung 12 dargestellt. Auf der Leiterplatte 20 sind zwei Teil-Heizelemente 22a, 22b angeordnet, die zusammen das Heizelement 22 bilden. Die Anwendungsfläche 18 ist orthogonal zu der Leiterplatte 20 an deren Stirnseite angeordnet. Durch die Anordnung der Teil-Heizelemente 22a, 22b in direktem Kontakt mit der Anwendungsfläche 18 wird eine gute thermische Kopplung erreicht. Die Rückseite der Anwendungsfläche 18 befindet sich in unmittelbarer räumlicher Nähe zur Leiterplatte 20. Die auf der Leiterplatte befindlichen Teil-Heizelemente sind vollautomatisch bestückbar. Um eine verbesserte thermische Kopplung zwischen den Teil-Heizelementen 22a, 22b und der Anwendungsfläche 18 zu erreichen, kann ein Wärmeleitmedium 26 zwischen der Anwendungsfläche 18 und dem Heizelement vorgesehen sein. Durch das Wärmeleitmedium 26 können Toleranzen ausgeglichen werden. Die in den Teil-Heizelementen 22a, 22b erzeugte Wärme wird an die Anwendungsfläche 18 weitergeleitet. Als Heizelement 22 bzw. als Teil-Heizelement 22a, 22b kann insbesondere ein Widerstand verwendet werden.

Die Teil-Heizelemente 22a, 22b sind im dargestellten Beispiel orthogonal zu der Stirnseite der Leiterplatte orientiert. Dies bewirkt eine vorteilhafte thermische Kopplung. Um das Heizelement 22 bzw. die Teil-Heizelemente 22a, 22b möglichst nahe an der Kante bzw. an der Stirnfläche der Leiterplatte 20 anzuordnen, kann eine geeignete Ausgestaltung der Kontaktierungsbereiche (Lötflächen oder Lötpads) auf der Leiterplatte 20 verwendet werden. Insbesondere können die Kontaktierungsbereiche so gewählt sein, dass eine Fläche des der Anwendungsfläche 18 zugewandten Kontaktierungsbereichs kleiner ist als eine Fläche des der Anwendungsfläche 18 abgewandten Kontaktierungsbereichs. Beispielsweise kann die Breite des Kontaktierungsbereichs an der Anwendungsfläche zwischen 0,5 und 0,75 der Breite des anderen Kontaktierungsbereichs betragen. Im Lötprozess zieht sich das Heizelement 22 dann in eine Position in unmittelbarer Nähe zur Stirnseite der Leiterplatte 20.

In besonders vorteilhafter Ausgestaltung kann weiterhin eine gemeinsame Erdung der Elemente auf der Leiterplatte an der Kante der Leiterplatte vorgesehen sein. Hierdurch wird eine elektrisch leitende Anwendungsfläche 18 geerdet.

In der Fig. 4 ist eine Ausführungsform der erfindungsgemäßen Vorrichtung 12 dargestellt. Zusätzlich zu den zwei Teil-Heizelementen 22a, 22b, die das Heizelement 22 bilden, ist auf der Leiterplatte 20 eine Prozessoreinheit 28, beispielsweise ein Mikrocontroller, angeordnet. Die Prozessoreinheit 28 kann dazu verwendet werden, die Teil-Heizelemente 22a, 22b jeweils einzeln zu steuern. Zudem können Messgrößen verarbeitet werden.

Weiterhin sind auf der Leiterplatte 20 im dargestellten Ausführungsbeispiel zwei Steuerelemente 30 angeordnet, die vorzugsweise als Transistoren ausgebildet sind und dazu dienen, eine Versorgungsspannung zu den Teil-Heizelementen 22a, 22b zu schalten. Die Steuerelemente 30 werden im dargestellten Ausführungsbeispiel von der Prozessoreinheit 28 angesteuert.

Ergänzend ist auf der Leiterplatte 20 ein Messelement 32 angeordnet. Als Messelement 32 kann insbesondere ein Temperatursensor verwendet werden, der in unmittelbarer Nähe zur Anwendungsfläche 18 positioniert ist, um die Temperatur der Anwendungsfläche 18 zu erfassen. Eine Auslesung des Messelements 32 kann ebenfalls durch die Prozessoreinheit 28 erfolgen.

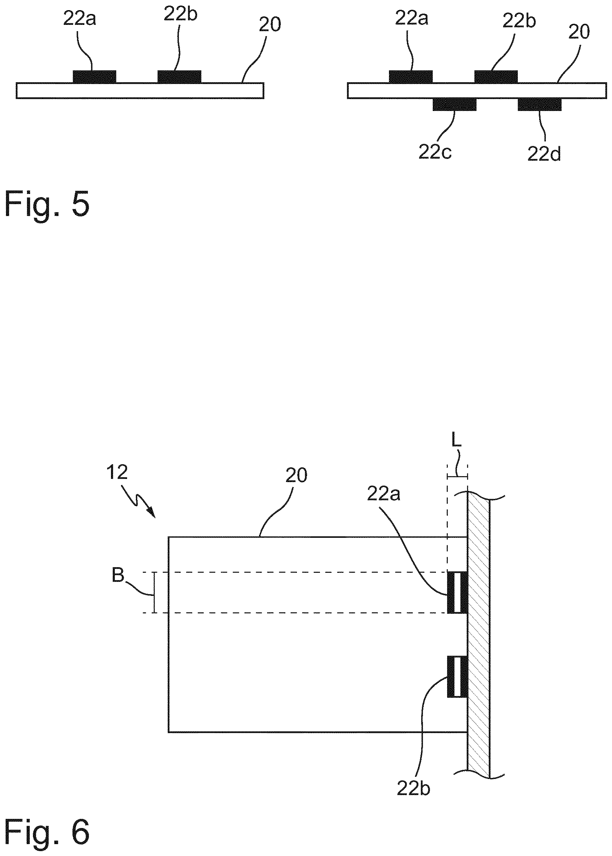

In der Fig. 5 sind zwei Schnittansichten der Leiterplatte 20 im Bereich der Stirnseite bzw. der Ankopplung an die Anwendungsfläche dargestellt. Auf der linken Seite sind zwei Teil-Heizelemente 22a, 22b einseitig auf der Leiterplatte 20 bestückt. Auf der rechten Seite sind insgesamt vier Teil-Heizelemente 22a, 22b, 22c, 22d auf beiden Seiten der Leiterplatte 20 bestückt.

Durch die versetzte Anordnung auf der rechten Seite wird erreicht, dass die an die Teil-Heizelemente 22a-22d anliegende Anwendungsfläche gleichmäßig erwärmt wird. Zudem wird ermöglicht, dass ein Temperaturgradient angelegt werden kann. Hierdurch ergibt sich eine optimierte Behandlung einer Hautirritation.

In der Fig. 6 ist schematisch eine Ausführungsform der erfindungsgemäßen Vorrichtung 12 dargestellt, bei der die zwei Teil-Heizelemente 22a, 22b auf der Leiterplatte 20 in gestauchter Bauform ausgebildet sind. Eine Ausdehnung in einer Länge L zwischen den Kontaktierungsstellen der Teil-Heizelemente 22a, 22b ist kleiner als eine Ausdehnung in einer Breite B orthogonal zu der Länge L. Die Teil-Heizelemente 22a, 22b liegen auf der Seite der größeren Ausdehnung an der Kante der Leiterplatte 20 an. Hierdurch kann die wirksame Heizfläche vergrößert werden und eine effiziente Erwärmung erreicht werden.

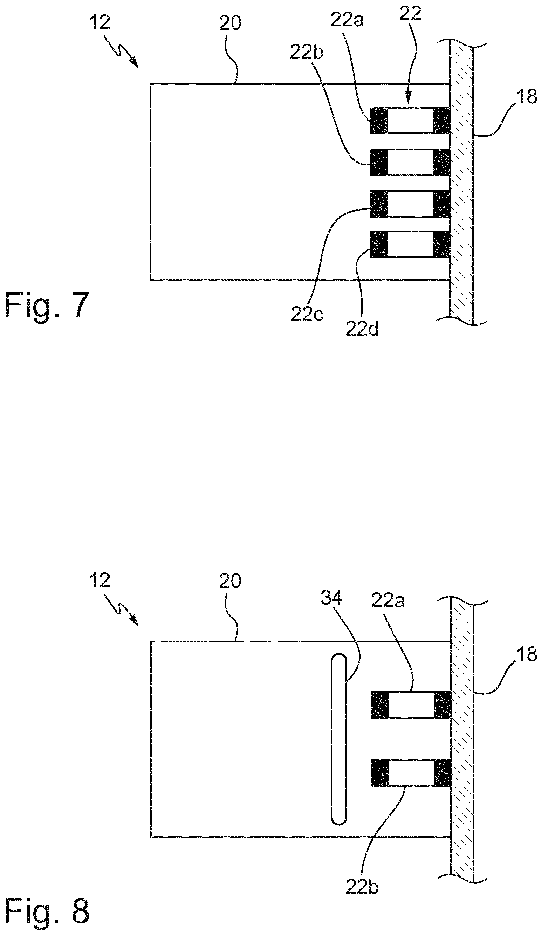

In der Fig. 7 ist schematisch eine erfindungsgemäße Vorrichtung 12 dargestellt, bei der ein Heizelement 22 mit insgesamt vier Teil-Heizelementen 22a, 22b, 22c, 22d auf einer Seite der Leiterplatte 20 angeordnet sind. Wenn die verschiedenen Teil-Heizelemente 22a-22d einzeln ansteuerbar sind, kann eine genaue Temperaturregelung realisiert werden. Ein Temperaturprofil auf der Anwendungsfläche 18 kann eingestellt werden.

In der Fig. 8 ist schematisch eine erfindungsgemäße Vorrichtung 12 dargestellt, bei der in der Leiterplatte 20 eine Aussparung 34 vorgesehen ist. Die Aussparung bewirkt, dass ein Wärmeabfluss über die Leiterplatte 20 weg von den Teil-Heizelementen 22a, 22b bzw. weg von der Anwendungsfläche 18 verringert wird. Durch eine mittels der Aussparung 34 erreichte thermische Entkopplung des der Anwendungsfläche 18 zugewandten Bereichs der Leiterplatte 20 kann die Dynamik im Sinne einer schnellen Erwärmung der Anwendungsfläche 18 und einer gesteigerten Nutzleistung des Systems verbessert werden.

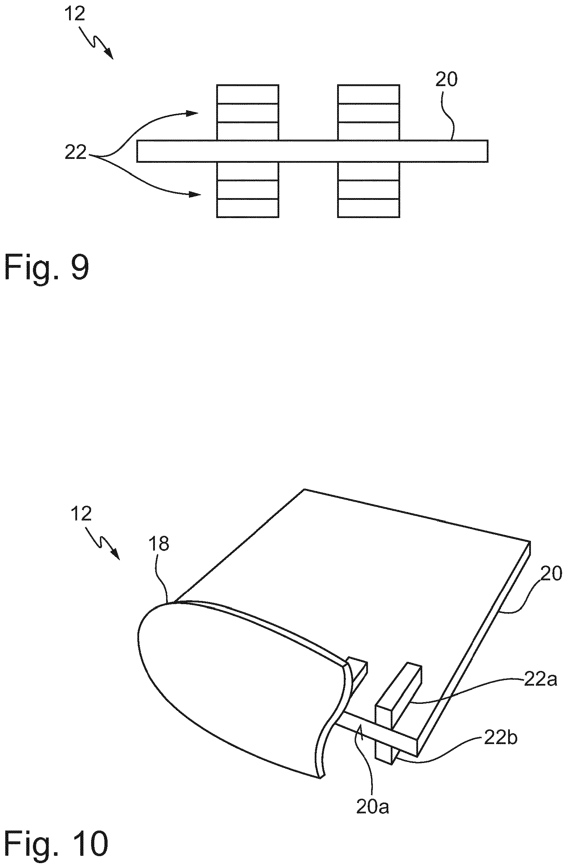

In der Fig. 9 ist schematisch eine Schnittansicht der erfindungsgemäßen Vorrichtung 12 im Bereich der Kopplung zwischen der Leiterplatte 20 und der Anwendungsfläche (nicht dargestellt) gezeigt. Insgesamt zwölf Teil-Heizelemente bilden zusammen das Heizelement 22. Durch eine derartige gestapelte Anordnung mehrerer Teil-Heizelemente kann eine wirksame Heizfläche vergrößert werden. Zudem wird eine thermische Belastung der einzelnen Elemente verringert.

In der Fig. 10 ist eine dreidimensionale Darstellung der erfindungsgemäßen Vorrichtung 12 mit geschnittener Anwendungsfläche 18 dargestellt. Die Teil-Heizelemente 22a, 22b sind beidseitig auf der Leiterplatte 20 bestückt. Die Stirnseite 20a der Leiterplatte 20 kann als metallisierte Leiterplattenkante ausgebildet sein, um hierdurch die thermische Kopplung zwischen Heizelement und Anwendungsfläche 18 zu optimieren.

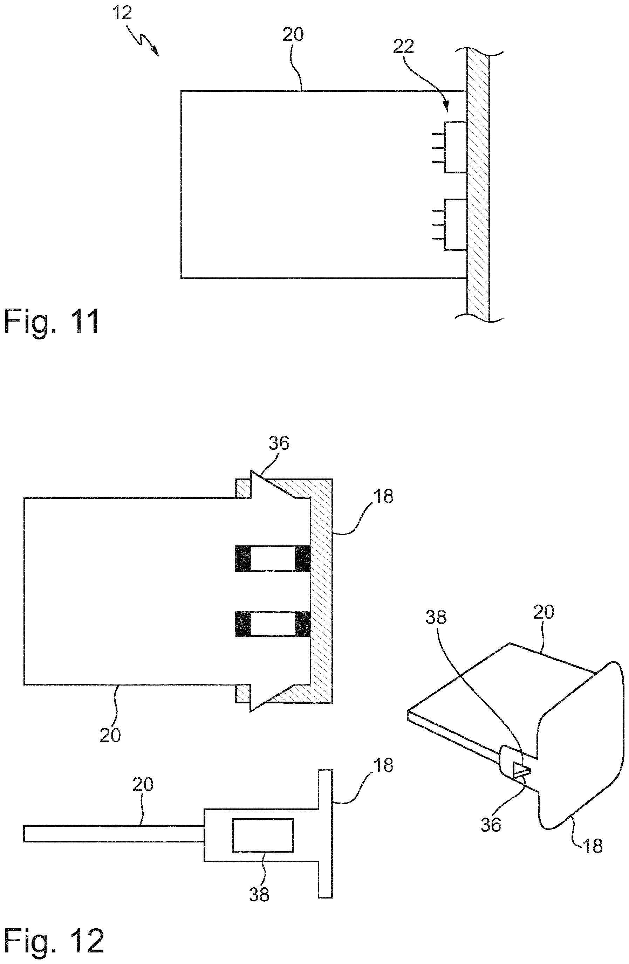

In der Fig. 11 ist schematisch eine Ausführungsform der erfindungsgemäßen Vorrichtung 12 mit einem alternativen Heizelement dargestellt. Insbesondere kommen als Heizelement unterschiedliche SMD-Bauteile in Frage. Beispielsweise können Halbleiterbauelemente oder andere Bauteile verwendet werden, bei denen eine Temperaturmessfunktion und/oder eine Sicherungsfunktion mit der Wärmeerzeugungsfunktion kombiniert sein können. Beispiele umfassen Transistoren, Mikrocontroller, PTC-Widerstände, NTC-Widerstände, etc.

In der Fig. 12 ist schematisch eine Befestigung der Anwendungsfläche 18 an der Leiterplatte 20 in drei verschiedenen Ansichten dargestellt. Für die Befestigung umfasst die Leiterplatte 20 ein erstes Befestigungselement 36, das als Haken ausgebildet ist. Die Anwendungsfläche 18 umfasst ein zweites Befestigungselement 38, das als Aussparung ausgebildet ist, in die der Haken eingreift. Durch die Verwendung der Befestigungselemente 36, 38 wird eine genaue und einfache Positionierung der Anwendungsfläche 18 an der Leiterplatte 20 erreicht. Es ergeben sich eine einfache Herstellbarkeit und ein robuster Aufbau.

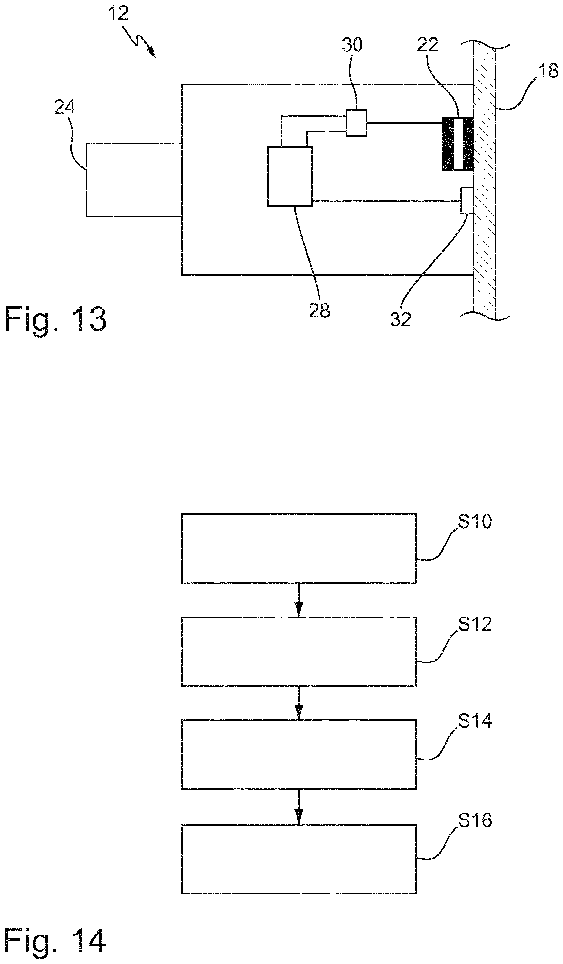

In der Fig. 13 ist schematisch eine Ausführungsform der erfindungsgemäßen Vorrichtung 12 dargestellt, bei der die Vorrichtung 12 eine als Stecker ausgebildete Energieschnittstelle 24 umfasst. Über den Stecker kann die Vorrichtung 12 mit einem Mobilgerät gekoppelt werden. Eine Biegebelastung auf einer Schnittstelle des Mobilgeräts wird durch eine orthogonale Ausrichtung der Anwendungsfläche 18 zu dem Stecker vermieden. Hierdurch wird eine hohe Lebensdauer erreicht. Im dargestellten Beispiel umfasst die Vorrichtung weiterhin ein Heizelement 22, eine Prozessoreinheit 28, ein Steuerelement 30 sowie ein Messelement 32.

In der Fig. 14 ist schematisch ein erfindungsgemäßes Verfahren zur thermisch-medizinischen Behandlung einer Hautoberfläche eines Menschen dargestellt. Das Verfahren umfasst Schritte des Empfangens S10 einer Nutzereingabe, des Ermittelns S12 einer gewünschten Behandlungstemperatur, des Erzeugens S14 von Steuerdaten und des Übermittelns S16 der Steuerdaten an eine Vorrichtung. Das erfindungsgemäße Verfahren kann insbesondere als Smartphone- oder Tablet-App implementiert sein. Über eine derartige App kann eine Ansteuerung der erfindungsgemäßen Vorrichtung 12 erfolgen. Ein Anwender kann basierend auf einer Nutzereingabe eine optimierte Wärmeeinbringung erreichen. Es ergibt sich eine komfortable Steuerungsmöglichkeit.

### Bezugszeichenliste

- 10: System
- 12: Vorrichtung
- 14: Mobilgerät
- 16: Geräteschnittstelle
- 18: Anwendungsfläche
- 20: Leiterplatte
- 22: Heizelement
- 24: Energieschnittstelle
- 26: Wärmeleitmedium
- 28: Prozessoreinheit
- 30: Steuerelement
- 32: Messelement
- 34: Aussparung
- 36: erstes Befestigungselement
- 38: zweites Befestigungselement

## Patentansprüche

1. Vorrichtung (12) zur thermisch-medizinischen Behandlung einer Hautoberfläche eines Menschen, mit:
einer Anwendungsfläche (18) mit einer Vorderseite zum Anlegen an die Hautoberfläche; und
einer Leiterplatte (20) mit einem darauf angeordneten Heizelement (22) zum Erwärmen der Anwendungsfläche (18) basierend auf einer Versorgungsspannung, wobei
die Anwendungsfläche (18) mit einer Rückseite an einer Stirnseite der Leiterplatte (20) in einem Winkel zu der Leiterplatte (20) angeordnet ist; und
das Heizelement (22) in einem Bereich der der Anwendungsfläche (18) zugewandten Stirnseite der Leiterplatte (20) auf der Leiterplatte (20) angeordnet ist und mit der Anwendungsfläche (18) thermisch gekoppelt ist,
wobei das Heizelement (22) an einem Rand der Leiterplatte (20) angeordnet ist.

2. Vorrichtung (12) nach Anspruch 1, wobei der Winkel zwischen Leiterplatte (20) und Anwendungsfläche (18) 90 Grad beträgt.

3. Vorrichtung (12) nach einem der vorstehenden Ansprüche, wobei
ein erster Kontaktierungsbereich des Heizelements (22) auf der Leiterplatte (20) der Anwendungsfläche (18) zugewandt ist und ein zweiter Kontaktierungsbereich des Heizelements (22) auf der Leiterplatte (20) der Anwendungsfläche (18) abgewandt ist; und
der erste Kontaktierungsbereich vorzugsweise kleiner als der zweite Kontaktierungsbereich ist.

4. Vorrichtung (12) nach einem der vorstehenden Ansprüche, wobei
das Heizelement (22) einen Heizwiderstand umfasst; und
eine Ausdehnung des Heizwiderstands in einer Länge (L) zwischen zwei Kontaktierungsseiten vorzugsweise kleiner ist als eine Ausdehnung in einer Breite des Heizelements (22) orthogonal zu der Länge.

5. Vorrichtung (12) nach einem der vorstehenden Ansprüche, wobei
die Anwendungsfläche (18) an der Stirnseite der Leiterplatte (20) anliegt; und
die Stirnseite der Leiterplatte (20) vorzugsweise metallisiert ist.

6. Vorrichtung (12) nach einem der vorstehenden Ansprüche, mit
einer Energieschnittstelle (24) zum reversiblen Ankoppeln der Vorrichtung (12) an ein Mobilgerät (14) und zum Empfangen der Versorgungsspannung von dem Mobilgerät (14), wobei
die Energieschnittstelle (24) bevorzugt als Mikro-USB, USB-C oder Lightning-Stecker ausgebildet ist und der Stecker sehr bevorzugt orthogonal zur Anwendungsfläche (18) ausgerichtet ist.

7. Vorrichtung (12) nach einem der vorstehenden Ansprüche, wobei
das Heizelement (22) mindestens zwei Teil-Heizelemente (22a, 22b, 22c, 22d) umfasst, die vorzugsweise identisch sind; und
die mindestens zwei Teil-Heizelemente (22a, 22b, 22c, 22d) vorzugsweise jeweils einzeln ansteuerbar sind, um ein Temperaturprofil an die Anwendungsfläche (18) anzulegen.

8. Vorrichtung (12) nach Anspruch 7, wobei
ein erstes Teil-Heizelement (22a, 22b, 22c, 22d) auf einer ersten Seite der Leiterplatte (20) angeordnet ist und ein zweites Teil-Heizelement (22a, 22b, 22c, 22d) auf einer zweiten Seite der Leiterplatte (20) angeordnet ist; und
das erste Teil-Heizelement (22a, 22b, 22c, 22d) und das zweite Teil-Heizelement (22a, 22b, 22c, 22d) vorzugsweise in ihrer Position entlang der Stirnseite der Leiterplatte (20) versetzt angeordnet sind.

9. Vorrichtung (12) nach einem der Ansprüche 7 bis 8, wobei ein unteres Teil-Heizelement (22a, 22b, 22c, 22d) und ein oberes Teil-Heizelement (22a, 22b, 22c, 22d) aufeinandergestapelt auf der Leiterplatte (20) angeordnet sind.

10. Vorrichtung (12) nach einem der vorstehenden Ansprüche, wobei die Leiterplatte (20) eine Aussparung (34) auf einer der Anwendungsfläche (18) abgewandten Seite des Heizelements (22) umfasst, um einen Wärmeabfluss zu vermindern.

11. Vorrichtung (12) nach einem der vorstehenden Ansprüche, mit:
einem auf der Leiterplatte (20) angeordneten Messelement (32) zum Ermitteln einer Temperatur der Anwendungsfläche (18), wobei das Messelement (32) in einem Bereich der der Anwendungsfläche (18) zugewandten Stirnseite der Leiterplatte (20) auf der Leiterplatte (20) angeordnet ist und mit der Anwendungsfläche (18) thermisch gekoppelt ist und vorzugsweise einen Temperatursensor umfasst;
einem auf der Leiterplatte (20) angeordneten Sicherungselement zum Überwachen einer Funktion des Heizelements (22), wobei das Sicherungselement vorzugsweise eine Schmelzsicherung umfasst;
einem auf der Leiterplatte (20) angeordneten Steuerelement (30) zum Steuern des Heizelements (22), wobei das Steuerelement (30) vorzugsweise einen Transistor zum Schalten der Versorgungsspannung umfasst; und/oder
einer auf der Leiterplatte (20) angeordneten Prozessoreinheit (28) zum Ansteuern des Heizelements (22), um eine Behandlungstemperatur der Anwendungsfläche (18) einzustellen.

12. Vorrichtung (12) nach einem der vorstehenden Ansprüche, mit:
einer auf der Leiterplatte (20) angeordneten Prozessoreinheit (28) zum Ansteuern des Heizelements (22), um eine Behandlungstemperatur der Anwendungsfläche (18) einzustellen; und
einer Steuerschnittstelle zum Empfangen von Steuerdaten mit Informationen über eine gewünschte Behandlungstemperatur, wobei
die Prozessoreinheit (28) zum Ansteuern des Heizelements (22) basierend auf den Steuerdaten ausgebildet ist; und
die Steuerschnittstelle vorzugsweise zum Empfangen der gewünschten Behandlungstemperatur von einer App eines Mobilgeräts (14) ausgebildet ist.

13. Vorrichtung (12) nach einem der vorstehenden Ansprüche, wobei
die Leiterplatte (20) ein erstes Befestigungselement (36) umfasst und die Anwendungsfläche (18) ein zweites Befestigungselement (38) umfasst, um die Anwendungsfläche (18) an der Leiterplatte (20) zu befestigen; und
eines der Befestigungselemente (36, 38) als Haken ausgebildet ist und das andere Befestigungselement (36, 38) als Aussparung ausgebildet ist, in die der Haken zum Befestigen der Anwendungsfläche (18) an der Leiterplatte (20) eingreifen kann.

14. System (10) zur thermisch-medizinischen Behandlung einer Hautoberfläche eines Menschen, mit:
einer Vorrichtung (12) nach einem der vorstehenden Ansprüche; und
einem Mobilgerät (14) mit einer Geräteschnittstelle (16) zum reversiblen Ankoppeln der Vorrichtung und zum Versorgen der Vorrichtung mit der Versorgungsspannung, wobei
die Geräteschnittstelle (16) vorzugsweise als Micro-USB, USB-C oder Lightning Anschluss ausgebildet ist.

15. Verfahren zur thermisch-medizinischen Behandlung einer Hautoberfläche eines Menschen, mit den Schritten:
Empfangen (S10) einer Nutzereingabe eines Nutzers eines Mobilgeräts (14);
Ermitteln (S12) einer gewünschten Behandlungstemperatur zum Anlegen an die Hautoberfläche;
Erzeugen (S14) von Steuerdaten basierend auf der gewünschten Behandlungstemperatur; und
Übermitteln (S16) der Steuerdaten an eine Vorrichtung (12) nach einem der Ansprüche 1 bis 13, die an das Mobilgerät angekoppelt ist.

## Claims

1. Device (12) for the thermal-medical treatment of a skin surface of a human being, comprising:
an application surface (18) having a front side for applying to the skin surface; and
a printed circuit board (20) having a heating element (22) arranged thereon for heating the application surface (18) based on a supply voltage, wherein
the application surface (18) is arranged with a back side on a face side of the printed circuit board (20) at an angle to the printed circuit board (20); and
the heating element (22) is arranged on the printed circuit board (20) in an area of the face side of the printed circuit board (20) facing the application surface (18) and is thermally coupled to the application surface (18),
wherein the heating element (22) is arranged at an edge of the printed circuit board (20).

2. Device (12) according to claim 1, wherein the angle between the printed circuit board (20) and the application surface (18) is 90 degrees.

3. Device (12) according to any one of the preceding claims, wherein
a first contacting area of the heating element (22) on the printed circuit board (20) faces the application surface (18) and a second contacting area of the heating element (22) on the printed circuit board (20) faces away from the application surface (18); and
the first contacting area is preferably smaller than the second contacting area.

4. Device (12) according to any one of the preceding claims, wherein
the heating element (22) comprises a heating resistor; and
an extension of the heating resistor in a length (L) between two contacting sides is preferably smaller than an extension in a width of the heating element (22) orthogonal to the length.

5. Device (12) according to any one of the preceding claims, wherein
the application surface (18) is adjacent to the face side of the printed circuit board (20); and
the face side of the printed circuit board (20) is preferably metallized.

6. Device (12) according to any one of the preceding claims, comprising
a power interface (24) for reversibly coupling the device (12) to a mobile device (14) and for receiving the supply voltage from the mobile device (14), wherein
the power interface (24) is preferably designed as a micro-USB, USB-C or Lightning connector and the connector is very preferably aligned orthogonally to the application surface (18).

7. Device (12) according to any one of the preceding claims, wherein
the heating element (22) comprises at least two partial heating elements (22a, 22b, 22c, 22d), which are preferably identical; and
the at least two partial heating elements (22a, 22b, 22c, 22d) are preferably each individually controllable in order to apply a temperature profile to the application surface (18).

8. Device (12) according to claim 7, wherein
a first partial heating element (22a, 22b, 22c, 22d) is arranged on a first side of the printed circuit board (20) and a second partial heating element (22a, 22b, 22c, 22d) is arranged on a second side of the printed circuit board (20); and
the first partial heating element (22a, 22b, 22c, 22d) and the second partial heating element (22a, 22b, 22c, 22d) are preferably arranged offset in their position along the face side of the printed circuit board (20).

9. Device (12) according to one of claims 7 to 8, wherein a lower partial heating element (22a, 22b, 22c, 22d) and an upper partial heating element (22a, 22b, 22c, 22d) are arranged stacked on top of each other on the printed circuit board (20).

10. Device (12) according to any one of the preceding claims, wherein the printed circuit board (20) comprises a recess (34) on a side of the heating element (22) facing away from the application surface (18) in order to reduce heat dissipation.

11. Device (12) according to any one of the preceding claims, comprising:
a measuring element (32) arranged on the printed circuit board (20) for determining a temperature of the application surface (18), wherein the measuring element (32) is arranged on the printed circuit board (20) in an area of the face side of the printed circuit board (20) facing the application surface (18) and is thermally coupled to the application surface (18) and preferably comprises a temperature sensor;
a fuse element arranged on the printed circuit board (20) for monitoring a function of the heating element (22), the fuse element preferably comprising a safety fuse;
a control element (30) arranged on the printed circuit board (20) for controlling the heating element (22), the control element (30) preferably comprising a transistor for switching the supply voltage; and/or
a processor unit (28) arranged on the printed circuit board (20) for controlling the heating element (22) in order to set a treatment temperature of the application surface (18).

12. Device (12) according to any one of the preceding claims, comprising:
a processor unit (28) arranged on the circuit board (20) for controlling the heating element (22) to set a treatment temperature of the application surface (18); and
a control interface for receiving control data with information about a desired treatment temperature, wherein
the processor unit (28) is designed to control the heating element (22) on the basis of the control data; and
the control interface is preferably designed to receive the desired treatment temperature from an app of a mobile device (14).

13. Device (12) according to any one of the preceding claims, wherein
the printed circuit board (20) comprises a first fixation element (36) and the application surface (18) comprises a second fixation element (38) for fixing the application surface (18) to the printed circuit board (20); and
one of the fixation elements (36, 38) is formed as a hook and the other fixation element (36, 38) is formed as a recess into which the hook can engage for fixing the application surface (18) to the printed circuit board (20).

14. System (10) for the thermal-medical treatment of a skin surface of a human being, comprising:
a device (12) according to any one of the preceding claims; and
a mobile device (14) with a device interface (16) for reversibly coupling the device and
for supplying the device with the supply voltage, wherein
the device interface (16) is preferably designed as a micro-USB, USB-C or Lightning connection.

15. Method for the thermal-medical treatment of a skin surface of a human being, comprising steps of
receiving (S10) a user input from a user of a mobile device (14);
determining (S12) a desired treatment temperature to be applied to the skin surface;
generating (S14) control data based on the desired treatment temperature; and
transmitting (S16) the control data to a device (12) according to any one of claims 1 to 13, which is coupled to the mobile device.

## Revendications

1. Dispositif (12) pour le traitement thermo-médical d'une surface de peau d'un être humain, comprenant :
une surface d'application (18) ayant une face avant destinée à être placée contre la surface de la peau ; et
une carte de circuit imprimé (20) ayant un élément chauffant (22) disposé sur celle-ci pour chauffer la surface d'application (18) sur la base d'une tension d'alimentation, dans laquelle
la surface d'application (18) étant disposée avec une face arrière sur une face frontale de la carte de circuit imprimé (20) à un angle par rapport à la carte de circuit imprimé (20) ; et
l'élément chauffant (22) étant disposé sur la carte de circuit imprimé (20) dans une zone de la face frontale de la carte de circuit imprimé (20) orientée vers la surface d'application (18) et étant couplé thermiquement à la surface d'application (18),
l'élément chauffant (22) étant disposé sur un bord de la carte de circuit imprimé (20).

2. Dispositif (12) selon la revendication 1, dans lequel l'angle entre la carte de circuit imprimé (20) et la surface d'application (18) est de 90 degrés.

3. Dispositif (12) selon l'une quelconque des revendications précédentes, dans lequel
une première zone d'établissement de contact de l'élément chauffant (22) sur la carte de circuit imprimé (20) fait face à la surface d'application (18) et une deuxième zone d'établissement de contact de l'élément chauffant (22) sur la carte de circuit imprimé (20) est opposée à la surface d'application (18) ; et
la première zone d'établissement de contact est de préférence plus petite que la deuxième zone d'établissement de contact.

4. Dispositif (12) selon l'une quelconque des revendications précédentes, dans lequel
l'élément chauffant (22) comprend une résistance chauffante ; et
une extension de la résistance chauffante dans une longueur (L) entre deux côtés d'établissement de contact est de préférence inférieure à une extension dans une largeur de l'élément chauffant (22) orthogonale à ladite longueur.

5. Dispositif (12) selon l'une quelconque des revendications précédentes, dans lequel
la surface d'application (18) est ajustée á la face avant de la carte de circuit imprimé (20) ; et
la face frontale de la carte de circuit imprimé (20) est de préférence métallisée.

6. Dispositif (12) selon l'une quelconque des revendications précédentes, comprenant
une interface d'énergie (24) pour le couplage réversible du dispositif (12) à un appareil mobile (14) et pour la réception de la tension d'alimentation de l'appareil mobile (14), dans lequel
l'interface d'énergie (24) est de préférence conçue comme un connecteur micro-USB, USB-C ou Lightning et le connecteur est de préférence orienté de manière orthogonale par rapport à la surface d'application (18).

7. Dispositif (12) selon l'une des revendications précédentes, dans lequel
l'élément chauffant (22) comprend au moins deux éléments chauffants partiels (22a, 22b, 22c, 22d), qui sont de préférence identiques ; et
les au moins deux éléments chauffants partiels (22a, 22b, 22c, 22d) peuvent être commandés, de préférence chacun individuellement, pour appliquer un profil de température à la surface d'application (18).

8. Dispositif (12) selon la revendication 7, dans lequel
un premier élément chauffant partiel (22a, 22b, 22c, 22d) est disposé sur un premier côté de la carte de circuit imprimé (20) et un deuxième élément chauffant partiel (22a, 22b, 22c, 22d) est disposé sur un deuxième côté de la carte de circuit imprimé (20) ; et
le premier élément chauffant partiel (22a, 22b, 22c, 22d) et le deuxième élément chauffant partiel (22a, 22b, 22c, 22d) sont de préférence décalés dans leur position le long de la face frontale de la carte de circuit imprim (20).

9. Dispositif (12) selon l'une des revendications 7 à 8, dans lequel un élément chauffant partiel inférieur (22a, 22b, 22c, 22d) et un élément chauffant partiel supérieur (22a, 22b, 22c, 22d) sont empilés les uns sur les autres sur la carte de circuit imprimé (20).

10. Dispositif (12) selon l'une quelconque des revendications précédentes, dans lequel la carte de circuit imprimé (20) comprend un évidement (34) sur un côté de l'élément chauffant (22) opposé à la surface d'application (18) afin de réduire l'écoulement de chaleur.

11. Dispositif (12) selon l'une quelconque des revendications précédentes, comprenant :
un élément de mesure (32) disposé sur la carte de circuit imprimé (20) pour déterminer une température de la surface d'application (18), l'élément de mesure (32) étant disposé sur la carte de circuit imprimé (20) dans une zone de la face frontale de la carte de circuit imprimé (20) orientée vers la surface d'application (18) et étant couplé thermiquement à la surface d'application (18) et comprenant de préférence un capteur de température ;
un élément de sécurité disposé sur la carte de circuit imprimé (20) pour surveiller un fonctionnement de l'élément chauffant (22), l'élément de sécurité comprenant de préférence un fusible ;
un élément de commande (30) disposé sur la carte de circuit imprimé (20) pour commander l'élément chauffant (22), l'élément de commande (30) comprenant de préférence un transistor pour commuter la tension d'alimentation ; et/ou
une unité de processeur (28) disposée sur la carte de circuit imprimé (20) pour commander l'élément chauffant (22) afin d'ajuster une température de traitement de la surface d'application (18).

12. Dispositif (12) selon l'une quelconque des revendications précédentes, comprenant :
une unité de processeur (28) disposée sur la carte de circuit imprimé (20) pour commander l'élément chauffant (22) afin d'ajuster une température de traitement de la surface d'application (18) ; et
une interface de commande pour recevoir des données de commande contenant des informations sur une température de traitement souhaitée, dans laquelle
l'unité de processeur (28) est conçue pour commander l'élément chauffant (22) sur la base des données de commande ; et
l'interface de commande est de préférence conçue pour recevoir la température de traitement souhaitée d'une application d'un appareil mobile (14).

13. Dispositif (12) selon l'une quelconque des revendications précédentes, dans lequel
la carte de circuit imprimé (20) comprend un premier élément de fixation (36) et la surface d'application (18) comprend un deuxième élément de fixation (38) pour fixer la surface d'application (18) à la carte de circuit imprimé (20) ; et
l'un des éléments de fixation (36, 38) est formé comme un crochet et l'autre élément de fixation (36, 38) est formé comme un évidement dans lequel le crochet peut s'engager pour fixer la surface d'application (18) à la carte de circuit imprimé (20).

14. Système (10) pour le traitement thermo-médical d'une surface de peau d'un être humain, comprenant :
un dispositif (12) selon l'une quelconque des revendications précédentes ; et
un appareil mobile (14) avec une interface d'appareil (16) pour le couplage réversible du dispositif et pour l'alimentation du dispositif avec la tension d'alimentation, dans lequel
l'interface d'appareil (16) est de préférence conçue comme une connexion micro-USB,
USB-C ou Lightning.

15. Procédé de traitement thermo-médical de la surface de la peau d'un être humain, comprenant les étapes suivantes :
réception (S10) d'une entrée d'utilisateur d'un utilisateur d'un appareil mobile (14) ;
déterminer (S12) une température de traitement souhaitée à appliquer à la surface de la peau ;
générer (S14) des données de commande sur la base de la température de traitement souhaitée ; et
transmettre (S16) les données de commande à un dispositif (12) selon l'une des revendications 1 à 13, couplé au dispositif mobile.
